# EUROPEAN PATENT SPECIFICATION

(11) **EP 1 573 329 B1**
(45) Date of publication and mention of the grant of the patent: **17.09.2008**
(21) Application number: 03812650.4
(22) Date of filing: 08.12.2003
(51) Int. Cl.: G01N 33/543, C12Q 1/68, G01N 27/00

(54) **BIOSENSOR WITH RF SIGNAL TRANSMISSION**
BIOSENSOR MIT RF-SIGNALÜBERTRAGUNG
SONDE BIOLOGIQUE A EMISSION DE SIGNAUX R.F.

(30) Priority: 09.12.2002 EP 02080173
(43) Date of publication of application: 14.09.2005
(73) Proprietor: Koninklijke Philips Electronics N.V., 5621 BA Eindhoven (NL)
(72) Inventor: KAHLMAN, Josephus, A., H., M., NL-5656 AA Eindhoven (NL); PRINS, Menno, W., J., NL-5656 AA Eindhoven (NL); VAN HOUTEN, Hendrik, NL-5656 AA Eindhoven (NL)
(74) Representative: Schouten, Marcus Maria
(86) International application number: PCT/IB2003/005786
(87) International publication number: WO 2004/053491

(56) References cited:
- WO-A-00/61803
- WO-A-00/66781
- DE-A- 2 221 371
- DE-A- 10 002 595
- US-A- 5 501 986
- US-A- 6 161 437
- US-A1- 2001 054 305

## Description

### BACKGROUND OF THE INVENTION

This invention relates to a device comprising a sensor element having biomolecular binding sites for a biomolecule and a method for detecting biomolecules in samples using such a device. Such devices are sometimes also called biosensors cartridges, the sensor elements are sometimes called biosensors. Biochips, biosensor chips, biological microchips, gene-chips or DNA chips are other words used to described such devices or sensors. In such a device a signal is caused by an interaction of the binding sites on a sensor surface with biochemical components in a fluid. Typically a fluid component binds specifically to molecules forming the bonding sites on a surface of the sensor element. The invention also relates to a method for determining the presence of or for measuring the amount ofbiomolecules using a biosensor device.

Biosensors have been used to determine the presence and/or the concentration of biomolecules in fluids. Examples ofbiomolecules are proteins, peptides, nucleic acids, carbohydrates and lipids. Examples of fluids are simple buffers and biological fluids, such as blood, serum, plasma, saliva, urine, tissue homogenates. The to be determined molecules are often also called the analyte..

In a biosensor cartridge a sensor element is provided with bonding sites. To facilitate detection, often markers or labels are used, e.g. small beads, nanoparticles or special molecules with fluorescent or magnetic properties. Labels can be attached before the analyte binds to the sensor, but also thereafter. Microparticles are sometimes used as a solid phase to capture the analyte. Solid phase microparticles can be made of a variety of materials, such as glass, plastic or latex, depending on the particular application. Some solid phase particles are made of ferromagnetic materials to facilitate their separation from complex suspensions or mixtures. The occurrence of a binding reaction, binding the solid phase microparticles (or some other marker which has captured the analyte) can be detected, e.g. by fluorescent markers.

The sensing of the molecules in the sensor element is called assay. Such assays may have various formats, e.g. simple binding, sandwich assay, competitive assay, displacement assay. In conventional solid-phase assays, the solid phase mainly aids in separating biomolecules that bind to the solid phase from molecules that do not bind to the solid phase. Separation can be facilitated by gravity, centrifugation, filtration, magnetism, flow-cytometry, microluidics, etc. The separation may be performed either in a single step in the assay or, more often, in multiple steps.

Often, it is desirable to perform two or more different assays on the same sample, in a single vessel and at about the same time. Such assays are known in the art as multiplex assays. Multiplex assays are performed to determine simultaneously the presence or concentration of more than one molecule in the sample being analyzed, or alternatively, to evaluate several characteristics of a single molecule, such as, the presence of several epitopes on a single protein molecule.

Biosensors are meant to be tools for doctors or laboratory personnel.

Measurement of a specific chemical reaction in the biosensor will lead to data that are to be interpreted by a certain apparatus. Due to the strict rules in the medical world the biosensor will be used only once. In other words: it must be cheap and simple. And, as with all things to be used in practice, operation is preferably easy.

An example of a device comprising a biosensor that can be relatively easily operated, is known from US 6,376,187. This device comprises an identification chip, that is powered by light and of which the memory is read out inductively. Independent thereof, the cartridge contains a biosensor, that is read out by means of fluorescence, i. e- a fluorescent marker binds with the analyte, which in its term binds at a bonding site and the presence of the flourescent marker at the bonding site, i. e. on the sensor element is detected by means of fluorescence, i. e. a fluorescent signal.

It is however a disadvantage of the known biosensor cartridge, that the sensitivity and correctness of the output is dependent on the strength of the fluorescent signal.

Thus, if an intermediate medium distorts the signal from the biosensor, the resulting measurement contains mistakes. And vice versa: if there is an output, it can only be trusted to a limited extent, since it contains an unknown, hardly or not controllable mistake due to the loss of intensity during the transfer of the signal from the biosensor to the reader.

WO 00/66781 discloses a rapid gene analysis architecture using a miniature spherical-shaped "ball" semiconductor device. An electron transfer detector is implemented having each molecular probe attached to a gate of a multi-gated FET which is fabricated on the ball. An electron acceptor complex is attached near the distal end of a short spacer chain composed of poly(ethylene oxide), linking the molecular probe to the ball- Multiple different molecular probes each containing an electron acceptor complex are placed on the ball. Target molecules containing electron donor complexes are passed over the ball surface allowing hybridization to occur. Following hybridization, the ball surface is then illuminated causing stimulation of the electron transfer from the donor complex to the acceptor complex located proximally to the gate of the multi-gated FET. The electron transfer reaction is detected by the FET at a site where hybridization has occurred producing an electron transfer reaction. Conversely, if there are probe-target mismatches, the observed electron transfer does not occur. A signal will only be generated for those sites where hybridization is complete. Electron transfer occurs simultaneously at all locations where there was complete hybridization. Hybridization information is then transmitted from the ball to a computer for processing.

DE 2,221,371 discloses a device for a wireless transmission of a measurement value from a transducer in an evaluation circuitry using inductively coupled oscillating circuits.

It is thus an object of the invention to provide a biosensor and a method that can be wirelessly operated and provides a more reliable signal.

### SUMMARY OF THE INVENTION

This object is achieved by the subject-matter of the independent claims.

According to an exemplary embodiment, a device as described in the opening paragraph is provided which comprises: a remote power transmission element, a resonance circuit, said resonance circuit comprising a biosensor as a resonance frequency determining sensor element, wherein binding at the bonding sites effects a physical property of the sensor element and thereby the resonance frequency, and a circuit for RF communication of an RF signal in dependence of the resonance frequency of the resonance circuit.

According to an exemplary embodiment, a method as described is provided Wherein a sensor device is used comprising a remote power transmission device, a resonance circuit comprising a biosensor as a resonance frequency determining sensor element, wherein binding at the bonding sites effects a physical property of the sensor element and thereby the resonance frequency, and a circuit for RF communication of an RF signal in dependence of the resonance frequency, the method comprising the steps of :
Binding a target to binding sites of the sensor element
Sending light to the photodiode for powering the biosensor device recording the RF signal emitted by the circuit for RF communication.

In a device in accordance with the invention a physical property or an output of the sensor element determines a resonance frequency in the resonance circuit. A binding reaction of the analyte (or a particle comprising the analyte, herein also called "the target") to a bonding site thus effects the resonance frequency (by effecting e. g. the L, the C, the R or the mass of the sensor). The change in the resonance frequency is used as a signal. This signal is recorded in the method of the invention. The selectivity is not, or at least much less than in the known devices, dependent on the intensity of the signal. Further more, the data conversion on the cartridge can be limited to a conversion of e. g. a change in e. g. an L, C, R value to a frequency change, which reduces the complexity of the device.
Systematic deviations of the resonance frequency of the resonance circuit can be circumvented easily, if necessary, by measurement of a calibration sample at the same time. Further advantages are: noise minimization can be effected easily by means of averaging over a longer time frame use can be made of impedance measurements, which measurements are in any case more sensitive than fluorescent measurements.

A remote power transmission element is a device which is powered remotely, it may e. g. be a photodiode, powered by light or a coil for power transmission of RF power.

A photodiode is preferred since it allows the provision of sufficient power(f. i. 0, 5V per photodiode). Besides, in comparison with the use of a coil for power transmission, it has the advantages that:
the necessary size of the photodiode is less than that of the inductor, thus minimizing surface of the chip, hence reducing costs for a power transmission with an inductor a larger power source in the reader is necessary.
the photodiode can be used as well for the transmission of signals to the device of the invention. For this aim, the same or one or more additional photodiodes may be used. The signals can be transmitted by modulation of the light. Alternatively, sensor elements of the device may be selectively activated through irradation with light from the photodiodes.

In case a coil is used the for power transmission or RF power, the remote power transmission device is tuned to a frequency different from the signal RF frequency to avoid interference between the power signal and the measurement signal.
It is remarked that electrical biosensors and devices are known. Such sensors measure a current (ΔI), voltage (ΔV), resistance (ΔR), or impedance (ΔZ).

Some examples of electrical biosensors are: Amperometric, Resistive (e.g. magnetoresistance,) Potentiometric, Impedimetric (e.g. magneto-impedance, capacitive), Calorimetric, Field-effect devices, Redox reaction devices and other.

These electronic biosensors are always galvanically coupled to a reader station.

There are several problems associated with galvanic contacts to a reader station:

Galvanic contacts are unreliable. In a clinical environment, biosensor equipment is washed and sterilized, which deteriorates the galvanic contacts and generates errors.

Galvanic contacts give ESD sensitivity.

Galvanic contacts require a relatively large pitch. This limits the number of contacts that can be made and unnecessarily increase the size and costs of the silicon chips.

Galvanic interfacing may require that conducting tracks are integrated in the cartridges, which complicates the device technology.

In the device in accordance with the invention the read-out is done via an RF signal, i.e. remotely, which removes the problems associated with galvanic couplings.

In respect of both types of known devices the sensitivity is greatly increased (by eliminating possible unreliabilities), while the complexity of the device is decreased.

The sensor element forms a part of the resonance circuit. This provides for a relatively simple configuration.

In such embodiments the sensor element may form a capacitor or a coil or a resistor within the resonance circuit.

Alternatively the sensor element forms part of a voltage or current supplying circuit, coupled to the resonance circuit, wherein the voltage or current of the supplying circuit is dependent on a physical property of the sensor element, and the resonance frequency of the resonance circuit is dependent on said voltage or current.

The invention further relates to a system of which the device of the invention is part and in which the method of the invention can be executed.

Such is a sytem for detecting biomolecules in samples provided on a biosensor device, which system comprises the biosensor device and a reader station comprising a power transmitting element for transmitting power to the biosensor device and an antenna and a receiver for receiving of signals to be wirelessly transmitted from the biosensor device to the reader station with a transmitting frequency. It is characterized in that the device of the invention is present. Furthermore, the apparatus comprises or is connected to an analyser for analysing the transmitting frequency of the signal of the biosensor device or the change thereof with respect to a calibration frequency. It is preferable that the system, and particularly the reader station comprises any means for processing said transmitting frequency and/or the change thereof. Such means is for instance a microprocessor, with which the signal can be converted into a digital format. In addition thereto, a suitable memory may be present. In such a memory the measuring data are preferably recorded with an identification number of the measured biosensor device. Furthermore, the reader station may comprise means for transmitting the resulting data, particularly a connection to a standard communication network, and/or means for displaying the results in the form of text or graphs.

These and other objects of the invention will be apparent from and elucidated with reference to the embodiments described hereinafter.

### BRIEF DESCRIPTION OF THE DRAWINGS

FIG. 1 is a schematic representation of a simple device of this invention.
FIG. 2 is a schematic representation of a layout of a device of this invention..
FIG. 3 schematically illustrates an electrical scheme for a device in accordance with the invention.
FIG. 4 is similar to fig. 3 but for the fact that the sensor element is by a capacitor.32.
FIG. 5 illustrates in more detail a part of fig. 4.
FIG. 6 illustrates an embodiment in which the sensor element forms a resistive element.
FIG. 7 illustrates an embodiment in which the sensor element(s) form(s) a GMR magnetoresistive element in a Wheatstone bridge configuration for supplying a voltage signal to a resonance circuit.
FIG. 8 schematically indicates a method in accordance with the invention.
FIG. 9 illustrates a multiarray device in accordance with the invention.
FIG. 10 illustrates an embodiment of the invention in which the remote power transmission element comprises a coil for receiving RF power whereby the remote power transmission element is arranged for receiving an RF frequency different from the resonance frequency.

In the different figures, the same reference numerals refer to the same or analogous elements unless otherwise indicated.

### DETAILED DESCRIPTION OF THE INVENTION

The present invention will be described with respect to a number of embodiments and with reference to certain drawings but the invention is not limited thereto.

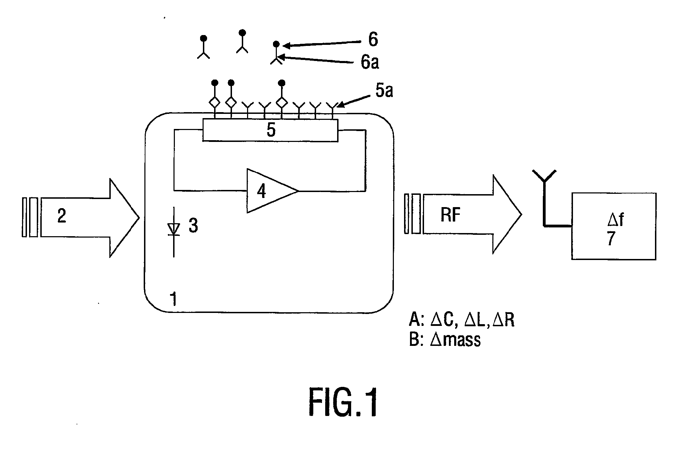

Fig. 1 is a schematic representation of the device and method in accordance with the invention. A biosensor cartridge 1 is provided with a photodiode 3 as a remote power transmission element. By shining light 2 on the photodiode the device is provided with power. The light may be visible light, UV or IR light, in an example the wavelength of the light is 780 nm. The device further comprises an oscillator circuit comprising in this example at least an amplifier 4 and a sensor element 5. The resonance frequency (eigenfrequency) is dependent on the properties of the elements forming the resonance circuit, in particular e.g. the capacitance, the inductance or the resistance of the sensor element within the oscillator circuit. Also the mass could have an influence on the oscillating frequency. The device 1 comprises a circuit (which may be the oscillator circuit itself or a larger entity comprising the oscillator circuit) for RF communication. The RF signal is dependent on the oscillator frequency of the oscillator circuit comprising the sensor element as one of its frequency determinative elements. In an example the RF frequency is e.g. 720 MHz. A surface or part of the sensor element 5 comprises bonding sites to which analytes 6a can bind. Generally labels or markers are used (beads 6 for instance) which bind to the binding sites 5a if and only if they comprise analytes 6a. Binding of the beads results in a change in a physical property of the sensor element (R,C,L) which in its turn changes the resonance frequency f of the resonance circuit, this can be done either directly, in case the sensor element forms a part of the resonance circuit per se as in this schematically indicated figure or, as in other examples, by means of a voltage- or current-generating circuit coupled to the resonance circuit, wherein the voltage or current of said circuit is dependent on a physical property of the sensor element, and in its turn determines the resonance frequency. The circuit for RF frequency emits a signal dependent on said change Δf (which signal could be a signal at the resonance frequency itself). This signal is emitted by the device 1 and received by receiver 7, which thus receives a signal comprising information on the change Δf in the resonance frequency f. This receiver 7 may have an analyser for analysing the change Δf or send the received signal to an analyser for analysing the change Δf. Any material not bounded to the bonding sites will have no or hardly no influence on the resonance frequency.

In the known device a fluorescence signal from the fluorescent markers is measured, i.e. the beads 6 are fluorescent. To this end light is shone on the fluorescent markers which are supposed to be on a binding site. However, if a fluorescent marker is not present on a binding site, but still in the light path (e.g. if it is not carefully washed away), or if other substances in the sample interfere in the light path (e.g. light scattering or absorption) there is a chance that an erroneous signal is produced. This contributes to the inaccuracy of the measurement. This becomes especially a problem if in one device many different analytes are to be measured. The fluorescent spectra of fluorescent markers are usually relatively broad and as a consequence in an assay in which several analytes are used, great care must be taken that cross-talk, i.e. a noise signal of a fluorescent signal of one analyte being present in the signal for the fluorescent marker for yet another analyte, does not occur. Even if such care is taken this will go at the expense of the speed of measurement. In the present invention, one or more of these problem are greatly reduced because the signal Δf is produced within the resonance circuit and any remaining markers outside the resonance circuit or not bounded on the surface will not influence the result. It is relatively easy to provide resonance circuits with clearly distinguishable resonance frequencies, making it more easy to distinguish one signal from another. This increases the accuracy of the measurement, as well as the speed with which the signals may be measured and thus the test results may be obtained.

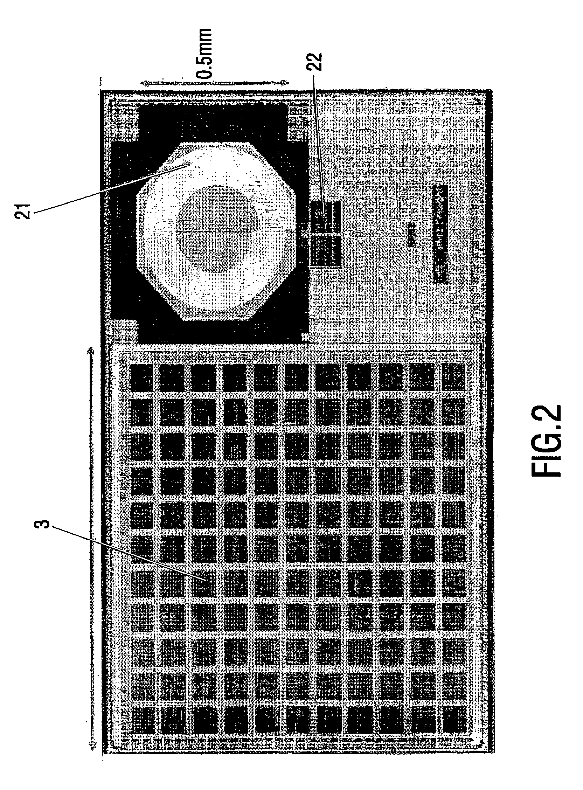

Figure 2 schematically shows a more true to life example of a device as shown in figure 1. The device comprises a photo diode 3, an on-chip conductor 21 and a capacitor 22.

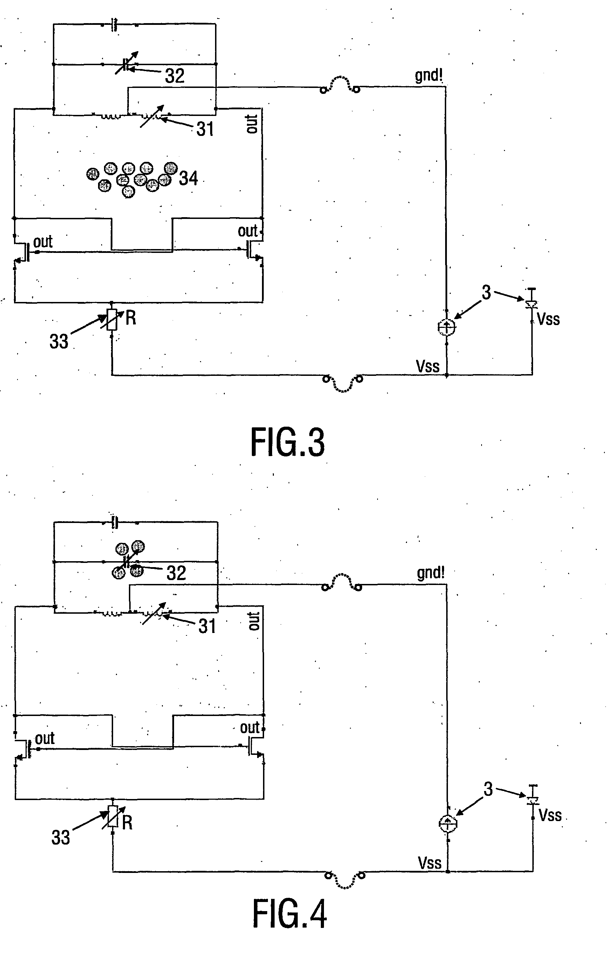

Figure 3 schematically illustrates an electrical representation of a device in accordance with the invention. The device comprises a photodiode 3, drawn here as photocurrent source Iₚₕ in parallel with diode D₀.

Schematically it is indicated that the oscillation circuit may comprise an inductance L (31), a capacitor C (32) and a resistive element R (33). The L, C and/or R value of these elements have an influence on the resonance frequency of the oscillator circuit. In different embodiments of a device in accordance with the invention, the sensor element may form a capacitor, a coil or a resistor within the resonance circuit. In this example it is schematically indicated that the sensor element forms an inductance L in the resonance circuit. Using magnetic beads 34 it is possible to change the L value of the coil. In this embodiment the sensor element would e.g. be a foil coil, i.e. a flat coil on a surface. The bonding sites would be present at or near the surface of the coil. The presence of the magnetic beads 34 at the bonding site and thus near the coil changes the L value of the coil and thereby changes the resonance frequency of the resonance circuit.
Some of the possible other arrangements are schematically shown in figures 4 to 8.

Figure 4 is similar to fig. 3. However, in this embodiment the sensor element is not formed by a coil, but by a capacitor.32. In this example the beads 35 are for instance beads with a relatively high dielectric constant. Binding at the binding sites will change the C value of the sensor element and thereby the resonance frequency of the oscillator.

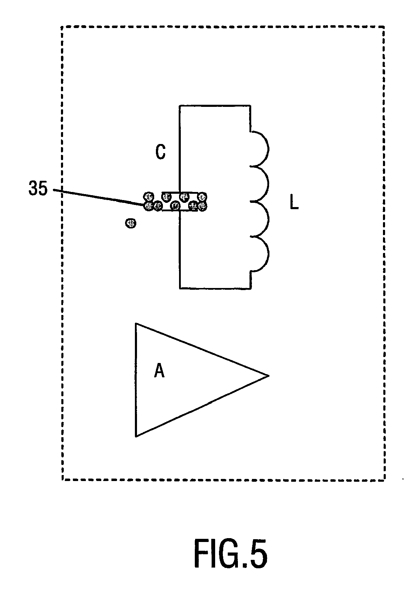

Figure 5 shows schematically details from figure 4. The resonance circuit is schematically indicated by the LC circuit and the amplifier A within the dotted-lined rectangle. The presence of the beads 35 in the capacitor C changes the capacitance of the capacitor and thereby the resonance frequency. In this figure, as in other figures, an amplifier A is schematically shown, as resonance circuits often have an amplifying part.

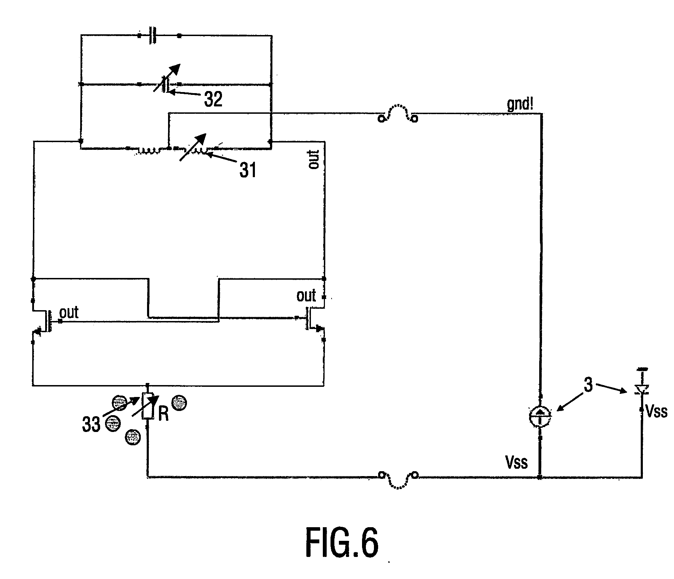

Figure 6 is similar to fig.3. However, in this embodiment the presence of electrically conductive beads at the resistance R changes the resistance value of said resistor and thereby the resonance frequency of the resonance circuit. A change in the resistance value of R will change the current going into the resonance circuit and thereby change the resonance frequency of the resonance circuit.

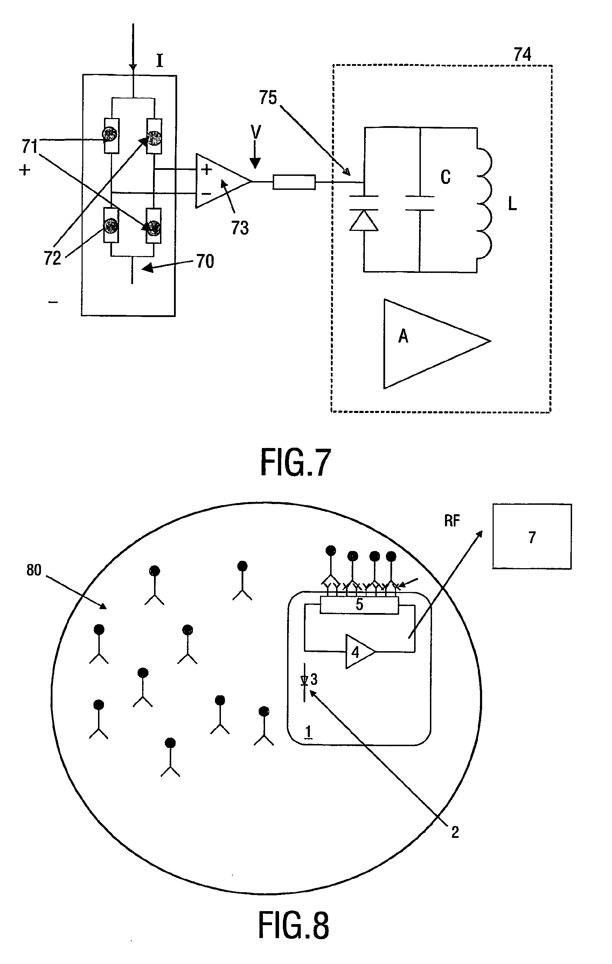

Figure 7 illustrates a variation on the scheme shown in figure 6. The biosensor comprises magnetoresistive detectors 71, 72 in a Wheatstone bridge configuration 70. The principles of magnetoresistive detection are for instance described D. R. Baselt,"A. biosensor based on magnetoresistance technology", Biosensors & Bioelectronics 13,731-739 (1998); in R. L. Edelstein et al.,"The BARC biosensor applied to the detection of biological warfare agents", Biosensors & Bioelectronics 14,805 (2000); and in M. M. Miller et al.,"A DNA array sensor utilizing magnetic microbeads and magnetoelectronic detection", Journal of Magnetism and Magnetic Materials 225(2001), pp.138-144. Suitable implementations are described in the non-prepublished documents EP 1 459 084 and EP 1 456 658. The resistance value R of one or more of the resistors is dependent on whether or not binding has taken place. Figure 7 illustrates a preferred embodiment in which the resistance of element 71 increase when binding takes place, indicated by the + sign in the figure, while for elements 72 the resistance decreases when binding takes place (indicated by the-sign). A Wheatstone bridge configuration is preferred since this allows for instance temperature dependence of the R values to be automatically compensated, at least when the same type of resistive elements is used in the bridge configuration. It is preferred to use this magnetoresistive detection in combination with modulation of an external magnetic field. Such modulation allows the separation of magnetic and non-magnetic contributions to the signal that is measured.

Magnetic labels are bonded to the sensor due to biochemical interactions. The labels are magnetised by an external magnetic field. The voltage from the Wheatstone bridge is dependent on the amount of magnetic labels located on the magnetoresistive sensors on the chip. The resonance frequency of the on-chip LC oscillator is modulated by this voltage. The set-up of the GMR sensors is optimised towards maximal signal at the output of amplifier 73.

The on-chip inductor (see figure 2) may act as an antenna for the RF signal it generates-The voltage V is amplified by amplifier 73 and send to a varicap diode 75 in a resonance circuit.

In a variation on this scheme the output I from the bio-sensors modulates the frequency of the LC oscillator.

Figure 8 schematically indicates a method in accordance with the invention and furthermore how a device in accordance with the invention may be used, In a vessel 80 a fluid having biomolecules is provided. To this fluid a marker is provided with binds the biomolecules. Thereafter a device (e. g. in the form of a chip) is provided having a sensor element with binding sites specific for the biomolecule. The biomolecules bind at the binding site at the sensor element. Light is shone on the chip, which emits in response an RF signal which is recorded by device 7. In this example the vessel is provided with only one chip, for simplicity sake. However, one of the great strengths of the devices and method in accordance with the invention is that many different chip (having sensor elements for various biomolecules) may be provided simultaneously and recorded simultaneously, as long as the resonance frequencies are distinguishable. This allows the presence and/or concentrations of a multitude of biomolecules to be measured simultaneously and accurately, which is a great advantage, it also allows concentrations of such biomolecules to be monitored, i. e. measured as a function of time simultaneously and accurately.

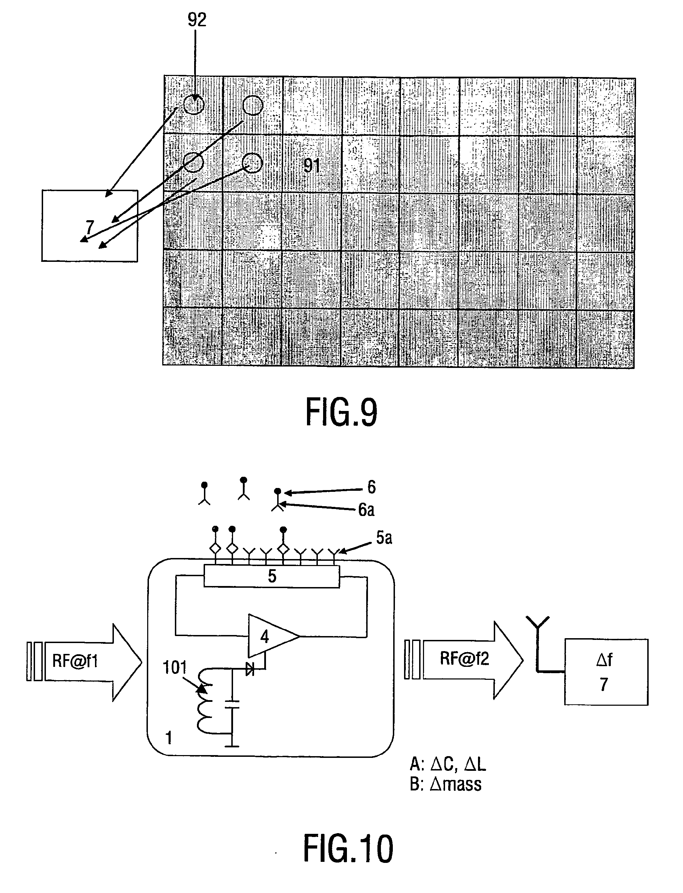

Figure 9 schematically illustrates a more complex device in accordance with the invention. In this device a large number of sub-devices 91, each in accordance with the invention is provided, at least some of which are for different biomolecules and emitting differing RF frequencies to a receiver 7. Each of the sub-devices has a fill opening 92. This multiarray enables to check for the many different biomocules simultaneously, accurately and fast.

It will be appreciated by persons skilled in the art that the present invention is not limited by what has been particularly shown and described hereinabove, but by the scope of the appended claims.

Reference numerals in the claims do not limit their protective scope.

Use of the verb "to comprise" and its conjugations does not exclude the presence of elements other than those stated in the claims. Use of the article "a" or "an" preceding an element does not exclude the presence of a plurality of such elements.

For instance, in the exemplary embodiments shown in figures 1 to 9 the remote power transmission element comprises or is constituted by a photodiode. Figure 10 shows an example of a device and method in accordance with the present invention in which the remote power transmission element comprises a coil 101 forming a part of an RF power receiving element which is arranged to receive power via an RF power signal at a frequency fl. This frequency differs from the RF frequency f2 of the oscillator. By using different frequency the power signal does not interfere with the measurement signal.

## Claims

1. A device (1) comprising a sensor element (5, 31, 32, 33, 71) having biomolecular binding sites (5a) for a biomolecule (6a), the device (1) comprising a remote power transmission element (3, 101), and being **characterised by** a resonance circuit, said resonance circuit comprising a biosensor as a resonance frequency determining sensor element (5, 31, 32), wherein binding at the binding sites (5a) effects a physical property (R, L, C, mass) of the sensor element (5, 31, 32, 33, 71) and thereby the resonance frequency (f), the resonance circuit being at least part of a circuit for RF communication of an RF signal (RF) in dependence of the resonance frequency of the resonance circuit.

2. A device as claimed in claim 1, **characterised in that** the remote power transmission element comprises a photodiode (3).

3. A device as claimed in claim 1, **characterised in that** the remote power transmission element comprises a coil (101) for receiving RF power whereby the remote power transmission element is arranged for receiving an RF frequency different from the resonance frequency.

4. A device as claimed in claim 1, **characterised in that** the sensor element (5, 31, 32) forms a part of the resonance frequency circuit.

5. A device as claimed in claim 4, **characterised in that** the sensor element (33, 71) forms part of a voltage or current supplying circuit, coupled to the resonance circuit, wherein the voltage (V) or current (I) of the supplying circuit is dependent on a physical property (R) of the sensor element, and the resonance frequency (f) of the resonance circuit is dependent on said voltage (V) or current (I).

6. A device as claimed in claim 1 or 4, **characterised in that** the sensor element (71) is a GMR magnetoresistive element.

7. A device as claimed in claim 3 or 4, **characterised in that** the sensor elements are resistive elements provided in a bridge configuration.

8. A device as claimed in claim 1, wherein the RF signal is a signal at the resonance frequency (f).

9. A method for detecting biomolecules in samples using a device (1) comprising a sensor element (5, 31, 32, 33, 71) having biomolecular binding sites (5a) for a biomolecule, wherein a sensor device is used, comprising a remote power transmission element (3), a resonance circuit comprising a biosensor as a resonance frequency determining sensor element (5, 31, 32), wherein binding at the bonding sites effects a physical property of the sensor element (5, 31, 32, 33, 71) and thereby the resonance frequency, the resonance circuit being at least part of a circuit for RF communication of an RF signal in dependence of the resonance frequency, the method comprising the steps of :
a) Binding a target to binding sites of the sensor element
b) Remotely sending power to the remote power transmission element for powering the biosensor device
c) recording the RF signal emitted by the circuit for RF communication.

10. A method as claimed in claim 9, **characterised in that** the remote power transmission element comprises a photodiode (3) and in step b light (2) is shone on the photodiode.

11. A method as claimed in claim 9, **characterised in that** the remote transmission element comprises a coil (101) for receiving RF power whereby the remote power transmission element is arranged for receiving an RF frequency different from the resonance frequency and in step b an RF frequency corresponding to the RF frequency of the remote power transmission element is emitted.

12. A system for detecting biomolecules in samples provided on a biosensor device, which system comprises the biosensor device and a reader station comprising a power transmitting element for transmitting power to the biosensor device and an antenna and a receiver for receiving of signals to be wirelessly transmitted from the biosensor device to the reader station with a transmitting frequency, **characterized in that**:
- a device as claimed in any of the Claims 1 to 8 is present,
- the apparatus comprises or is connected to an analyser for analysing the transmitting frequency of the signal of the biosensor device or the change thereof with respect to a calibration frequency.

## Patentansprüche

1. Einrichtung (1) mit einem Sensorelement (5, 31, 32, 33, 71) mit biomolekularen Bindungsstellen (5a) für ein Biomolekül (6a), wobei die Einrichtung (1) ein abgesetztes Energieübertragungselement (3, 101) umfasst und **gekennzeichnet ist durch** einen Resonanzkreis, wobei der genannte Resonanzkreis einen Biosensor als einen die Resonanzfrequenz bestimmendes Sensorelement (5, 31, 32) umfasst, in dem das Ankoppeln an die Bindungsstellen (5a) eine physikalische Eigenschaft (R, L, C, Masse) des Sensorelements (5, 31, 32, 33, 71) beeinflusst und **dadurch** die Resonanzfrequenz (f), wobei der Resonanzkreis zumindest Teil einer Schaltung zur HF-Kommunikation eines HF-Signals (HF) in Abhängigkeit von der Resonanzfrequenz des Resonanzkreises ist.

2. Einrichtung nach Anspruch 1, **dadurch gekennzeichnet, dass** das abgesetzte Energieübertragungselement eine Fotodiode (3) umfasst.

3. Einrichtung nach Anspruch 1, **dadurch gekennzeichnet, dass** das abgesetzte Energieübertragungselement eine Spule (101) zum Empfangen von HF-Energie umfasst, wobei das abgesetzte Energieübertragungselement dafür ausgelegt ist, eine HF-Frequenz zu empfangen, die sich von der Resonanzfrequenz unterscheidet.

4. Einrichtung nach Anspruch 1, **dadurch gekennzeichnet, dass** das Sensorelement (5, 31, 32) einen Teil des Resonanzfrequenzschaltkreises bildet.

5. Einrichtung nach Anspruch 4, **dadurch gekennzeichnet, dass** das Sensorelement (33, 71) Teil einer mit dem Resonanzkreis gekoppelten Spannungs- oder Stromversorgungsschaltung bildet, wobei die Spannung (V) oder der Strom (I) der Versorgungsschaltung von einer physikalischen Eigenschaft (R) des Sensorelements abhängt und die Resonanzfrequenz (f) des Resonanzkreises von der genannten Spannung (V) oder dem genannten Strom (I) abhängt.

6. Einrichtung nach Anspruch 1 oder 4, **dadurch gekennzeichnet, dass** das Sensorelement (71) ein GMR-Element ist.

7. Einrichtung nach Anspruch 3 oder 4, **dadurch gekennzeichnet, dass** die Sensorelemente widerstandsbehaftete Elemente in einer Brückenkonfiguration sind.

8. Einrichtung nach Anspruch 1, wobei das HF-Signal ein Signal mit Resonanzfrequenz (f) ist.

9. Verfahren zum Erkennen von Biomolekülen in Proben mit Hilfe einer Vorrichtung (1) mit einem Sensorelement (5, 31, 32, 33, 71) mit biomolekularen Bindungsstellen (5a) für ein Biomolekül, wobei eine Sensoreinrichtung verwendet wird, umfassend ein abgesetztes Energieübertragungselement (3), einen Resonanzkreis mit einem Biosensor als ein die Resonanzfrequenz bestimmendes Sensorelement (5, 31, 32), in dem das Ankoppeln an die Bindungsstellen eine physikalische Eigenschaft des Sensorelements (5, 31, 32, 33, 71) beeinflusst und **dadurch** die Resonanzfrequenz, wobei der Resonanzkreis zumindest Teil einer Schaltung zur HF-Kommunikation eines HF-Signals in Abhängigkeit von der Resonanzfrequenz ist, wobei das Verfahren die folgenden Schritte umfasst:
a) Ankoppeln eines Ziels an die Bindungsstellen des Sensorelements,
b) Senden von Energie an das abgesetzte Energieübertragungselement aus der Ferne, um die Biosensor-Einrichtung mit Energie zu versorgen,
c) Aufzeichnen des durch die Schaltung zur HF-Kommunikation emittierten HF-Signals.

10. Verfahren nach Anspruch 9, **dadurch gekennzeichnet, dass** das abgesetzte Energieübertragungselement eine Fotodiode (3) umfasst und in Schritt b die Fotodiode mit Licht (2) bestrahlt wird.

11. Verfahren nach Anspruch 9, **dadurch gekennzeichnet, dass** das abgesetzte Übertragungselement eine Spule (101) zum Empfangen von HF-Energie umfasst, wobei das abgesetzte Energieübertragungselement dafür ausgelegt ist, eine HF-Frequenz zu empfangen, die sich von der Resonanzfrequenz unterscheidet, und wobei in Schritt b eine HF-Frequenz emittiert wird, die der HF-Frequenz des abgesetzten Energieübertragungselements entspricht.

12. System zum Erkennen von Biomolekülen in auf einer Biosensoreinrichtung vorgelegten Proben, wobei das System die Biosensoreinrichtung und eine Lesestation mit einem Energieübertragungselement zum Übertragen von Energie an die Biosensoreinrichtung und eine Antenne und einen Empfänger zum Empfangen von drahtlos von der Biosensoreinrichtung zur Lesestation mit einer Sendefrequenz zu übertragenden Signalen umfasst, **dadurch gekennzeichnet, dass**
- eine Vorrichtung nach einem der Ansprüche 1 bis 8 vorhanden ist,
- das Gerät einen Analysator zum Analysieren der Sendefrequenz des Signals der Biosensoreinrichtung oder deren Änderung in Bezug auf eine Kalibrierfrequenz umfasst oder hiermit verbunden ist.

## Revendications

1. Dispositif (1) comprenant un élément de capteur (5, 31, 32, 33, 71) ayant des sites de liaison biomoléculaires (5a) pour une biomolécule (6a), le dispositif (1) comprenant un élément de télétransmission de puissance (3, 101) et étant **caractérisé par** un circuit de résonance, ledit circuit de résonance comprenant un biocapteur en tant qu'un élément de capteur (5, 31, 32) déterminant la fréquence de résonance dans lequel la liaison aux sites de liaison (5a) effectue une propriété physique (masse R, L, C) de l'élément de capteur (5, 31, 32, 33, 71) et de ce fait la fréquence de résonance (f), le circuit de résonance étant au moins une partie du circuit pour la communication de radiofréquence d'un signal de radiofréquence (RF) en fonction de la fréquence de résonance du circuit de résonance.

2. Dispositif selon la revendication 1, **caractérisé en ce que** l'élément de télétransmission de puissance comprend une photodiode (3).

3. Dispositif selon la revendication 1, **caractérisé en ce que** l'élément de télétransmission de puissance comprend une bobine (101) pour recevoir une puissance de radiofréquence, cas dans lequel l'élément de télétransmission de puissance est agencé de manière à recevoir une fréquence RF qui est différente de la fréquence de résonance.

4. Dispositif selon la revendication 1, **caractérisé en ce que** l'élément de capteur (5, 31, 32) fait partie du circuit de fréquence de résonance.

5. Dispositif selon la revendication 4, **caractérisé en ce que** l'élément de capteur (33, 71) fait partie d'un circuit d'alimentation en tension ou en courant qui est couplé au circuit de résonance, dans lequel la tension (V) ou le courant (I) du circuit d'alimentation est dépendant d'une propriété physique (R) de l'élément de capteur et dans lequel la fréquence de résonance (f) du circuit de résonance est dépendante de ladite tension (V) ou dudit courant (I).

6. Dispositif selon la revendication 1 ou 4, **caractérisé en ce que** l'élément de capteur (71) est un élément de magnétorésistance géante (GMR).

7. Dispositif selon la revendication 3 ou 4, **caractérisé en ce que** les éléments de capteur sont des éléments de résistance qui sont prévus dans une configuration en pont.

8. Dispositif selon la revendication 1, dans lequel le signal de radiofréquence est un signal à la fréquence de résonance (f).

9. Procédé pour détecter des biomolécules dans des échantillons à l'aide d'un dispositif (1) comprenant un élément de capteur (5, 31, 32, 33, 71) ayant des sites de liaison biomoléculaires (5a) pour une biomolécule, dans lequel on utilise un dispositif de capteur comprenant un élément de télétransmission de puissance (3), un circuit de résonance comprenant un biocapteur en tant qu'un élément de capteur (5, 31, 32) déterminant la fréquence de résonance dans lequel la liaison aux sites de liaison effectue une propriété physique de l'élément de capteur (5, 31, 32, 33, 71) et de ce fait la fréquence de résonance, le circuit de résonance étant au moins une partie d'un circuit pour la communication de radiofréquence d'un signal de radiofréquence (RF) en fonction de la fréquence de résonance, le procédé comprenant les étapes consistant à:
a) lier une cible aux sites de liaison de l'élément de capteur;
b) envoyer à distance une puissance à l'élément de télétransmission de puissance pour la mise sous tension du dispositif de biocapteur;
c) enregistrer le signal de radiofréquence qui est émis par le circuit pour la communication de radiofréquence.

10. Procédé selon la revendication 9, **caractérisé en ce que** l'élément de télétransmission de puissance comprend une photodiode (3) et dans l'étape b une lumière (2) est rayonnée sur la photodiode.

11. Procédé selon la revendication 9, **caractérisé en ce que** l'élément de télétransmission de puissance comprend une bobine (101) pour recevoir une puissance de radiofréquence, cas dans lequel l'élément de télétransmission de puissance est agencé de manière à recevoir une fréquence RF qui est différente de la fréquence de résonance et dans l'étape b une fréquence RF correspondant à la fréquence RF de l'élément de télétransmission de puissance est émise.

12. Système pour détecter des biomolécules dans des échantillons qui sont prévus sur un dispositif de biocapteur, lequel système comprend le dispositif de biocapteur et une station de lecteur comprenant un élément de transmission de puissance pour transmettre la puissance au dispositif de biocapteur et une antenne et un récepteur pour la réception de signaux à transmettre sans fil à partir du dispositif de biocapteur à la station de lecteur avec une fréquence de transmission, **caractérisé en ce que**:
- il est présent un dispositif, tel que revendiqué dans l'une quelconque des revendications précédentes 1 à 8,
- l'appareil comprend ou est connecté à un analyseur pour analyser la fréquence de transmission du signal du dispositif de capteur ou le changement de celui-ci par rapport à une fréquence d'étalonnage.
